(19) Europäisches Patentamt / European Patent Office / Office européen des brevets

(11) **EP 1 870 067 A1**

(12) **EUROPEAN PATENT APPLICATION**
published in accordance with Art. 153(4) EPC

(43) Date of publication:
**26.12.2007 Bulletin 2007/52**

(51) Int Cl.:
*A61F 13/49* (2006.01)   *A61F 13/53* (2006.01)
*A61F 13/15* (2006.01)   *A61F 13/539* (2006.01)
*A61F 13/494* (2006.01)   *A61F 13/472* (2006.01)

(21) Application number: **06729911.5**

(22) Date of filing: **24.03.2006**

(86) International application number:
**PCT/JP2006/305963**

(87) International publication number:
**WO 2006/109524 (19.10.2006 Gazette 2006/42)**

(84) Designated Contracting States:
**DE FR GB SE**

(30) Priority: **01.04.2005   JP   2005106897**

(71) Applicant: **Kao Corporation**
**Chuo-ku**
**Tokyo 103-8210 (JP)**

(72) Inventors:
• **KASAI, Takao,**
**c/o Kao Corporation**
**Haga-gun,**
**Tochigi 3213497 (JP)**

• **KOUTA, Takuya,c/o Kao Corporation**
**Haga-gun, Tochigi 3213497 (JP)**
• **KATOH, Takahiro,**
**c/o Kao Corporation**
**Haga-gun,**
**Tochigi 3213497 (JP)**
• **NIINOMI, Masahiko,**
**c/o Kao Corporation**
**Haga-gun,**
**Tochigi 3213497 (JP)**

(74) Representative: **Vossius & Partner**
**Siebertstrasse 4**
**81675 München (DE)**

(54) **ABSORBENT ARTICLE**

(57)     An absorbent article 10 has an absorbent member 1. The absorbent member 1 includes a web 2 of hydrophilic continuous fibers and a superabsorbent polymer 3 embeddedly supported in the web 2. The superabsorbent polymer 3 has its particle size varied between the topsheet side and the backsheet side of the web 2, preferably has a larger size in the topsheet side than in the backsheet side of the web 2. The superabsorbent polymer preferably has a rate of liquid permeation of 150 to 1500 g/min under a load of 0.6 kPa. The continuous fibers are preferably crimped fibers with a crimp percentage of 10% to 90%.

## Fig.1

EP 1 870 067 A1

## Description

Technical Field

The present invention relates to an absorbent article such as a disposable diaper, a sanitary napkin, and an incontinence pad.

Background Art

Absorbent members for absorbent articles using an opened tow of continuous filaments are known. Included is an absorbent member comprising a crimped cellulose acetate fiber tow layer and a ground pulp layer superposed on one side of the tow layer, the two layers being united by pressing in the thickness direction (see JP 57-160457A). The absorbent member is described as having improved body fluid distribution. However, because cellulose acetate fiber is inferior to pulp in water absorption, a large quantity of ground pulp should be used in combination to secure increased absorption capacity. As a result, the absorbent member has an increased thickness, which reduces wearing comfort of the absorbent article.

WO 2001/034082 proposes an absorbent core composed of an upper layer, a lower layer, and an absorbent layer interposed therebetween. The absorbent layer includes a layer of spread superabsorbent polymer particles on which a fiber layer made of a cellulose acetate fiber tow is disposed. The superabsorbent polymer has a part thereof bonded to the lower layer with an adhesive and another part thereof entering the fiber tow layer. In above absorbent core, although part of the superabsorbent polymer enters the fiber tow layer, the most part of the superabsorbent polymer is bonded to the lower layer. In other words, the fiber tow layer and the superabsorbent polymer layer are independent of each other so that the structure of the absorbent core tends to be delaminated in its thickness direction when deformed during use by the wearer's movement.

Furthermore, the superabsorbent polymer bonded to the lower layer can be hindered from absorbing liquid by the influence of the adhesive. This results in reduced absorption efficiency per unit weight of the superabsorbent polymer. To increase the absorption capacity cannot be achieved without increasing the amount of the superabsorbent polymer to be used more than necessary. Hence, it is demanded to develop a method of loosely fix the superabsorbent polymer in order to make efficient use of the superabsorbent polymer.

According to WO 2001/034082, slight absorption swelling of the superabsorbent polymer greatly increases the pressure inside the absorbent member, making the superabsorbent polymer incapable of further swelling. As a result, the part of the absorbent member that is sufficiently contributory to absorption is limited to the central portion, and the absorbent member fails to maintain high absorptivity.

Among known absorbent layers having a tow of continuous filaments is the one disclosed in JP 2001-276125A, in which the tow extends in the thickness direction of the absorbent layer. The publication says that excrement moves downward through the interstices in the tow and draws away from the wearer's skin and is thus prevented from causing overhydration or rash. In order for the absorbent layer to have such a structure, the tow should have some length so that the absorbent layer is of necessity thick.

Disclosure of the Invention

The present invention provides an absorbent article having an absorbent member. The absorbent member has a web of hydrophilic continuous fibers and a superabsorbent polymer embeddedly supported in the web. The superabsorbent polymer has its particle size varied between the topsheet side and the backsheet side of the web.

Brief Description of the Drawings

Fig. 1 schematically illustrates the structure of an absorbent member in one embodiment of the absorbent article according to the present invention.
Fig. 2 is a plan of a flat type disposable diaper as an embodiment of the absorbent article according to the present invention.
Fig. 3 is a cross-section taken along line segment III-III in Fig. 2.
Fig. 4 is an enlarged fragmentary view of the diaper of Fig. 2 being worn (in a relaxed state).
Fig. 5 is a fragmentary cross-section of a modification of the diaper shown in Fig. 2 (corresponding to Fig. 3).
Fig. 6 is a fragmentary cross-section of another modification of the diaper shown in Fig. 2 (corresponding to Fig. 3).
Fig. 7 is an exploded perspective of a pull-on diaper as another embodiment of the absorbent article according to the present invention.

Fig. 8 is a fragmentary, transverse cross-section of the crotch portion of the absorbent assembly in the diaper shown in Fig. 7.

Fig. 9 is a fragmentary, transverse cross-section of the crotch portion of the absorbent assembly in a modification of the diaper shown in Fig. 7 (corresponding to Fig. 8).

Fig. 10 is a schematic view illustrating another embodiment of the absorbent member in the absorbent article according to the present invention (corresponding to Fig. 1).

Detailed Description of the Invention

The present invention will be described based on its preferred embodiments with reference to the accompanying drawings. The absorbent article of the present invention primarily contemplates use for absorbing and retaining discharged body fluids such as urine and menstrual blood. The absorbent article of the invention is exemplified by disposable diapers, sanitary napkins, and incontinence pads, but is not limited thereto, and a wide range of articles usable to absorb fluids discharged from a human body are included.

The absorbent article of the present invention typically includes a topsheet, a backsheet, and a liquid retentive absorbent member interposed between the two sheets. The topsheet and backsheet can be of any material commonly employed in the art with no particular restriction. For example, useful topsheets are liquid permeable sheets made of hydrophilized nonwoven fabrics or perforated films. Useful backsheets are liquid impermeable or water repellent sheets such as a thermoplastic resin film or a laminate of the film and a nonwoven fabric. The backsheet may have water vapor permeability. The absorbent article can have other members configured for specific purposes of specific articles. Such other members are well known to those skilled in the art. For example, an absorbent article for application to a disposable diaper or a sanitary napkin may have one or more pairs of barrier cuffs on both lateral side portions of the topsheet.

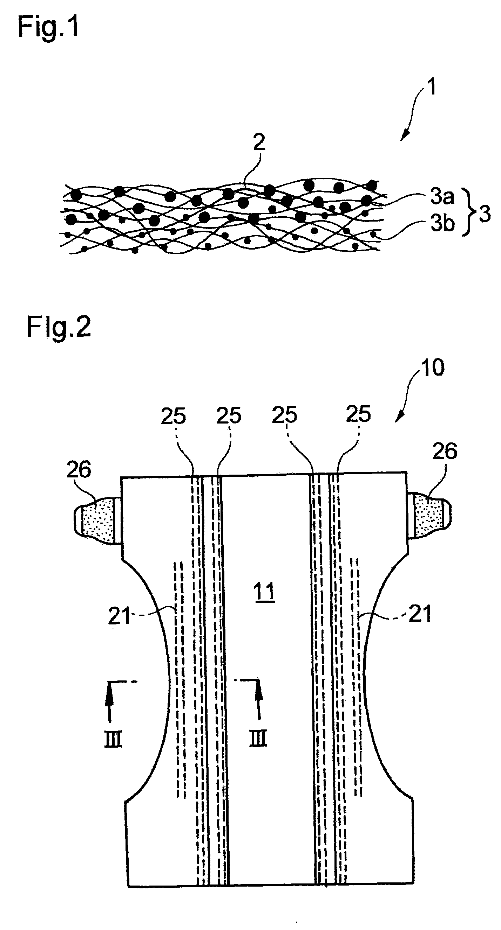

Fig. 1 is a schematic illustration of an absorbent member for an embodiment of the absorbent article according to the present invention. The absorbent member 1 of this embodiment is characterized by high rate of absorption. It is also characterized by having sufficient absorption capacity and yet being thin and lightweight. The thus characterized absorbent member 1 includes a continuous fiber web (hereinafter simply referred to as a web) 2 and superabsorbent polymer particles 3 embeddedly supported in the web 2. In Fig. 1, the upper side is a topsheet side, i.e., a side to face the topsheet, and the lower side is a backsheet side, i.e., a side to face the backsheet.

The term "topsheet side" as used herein is intended to include the portion of an absorbent member from the topsheet facing surface to half the thickness of the absorbent member. The term "particle size in the topsheet side" denotes the average particle size of the superabsorbent polymer particles present in that portion as measured on a cut area of the absorbent member under stereomicroscopic observation at a magnification of 10 times. To prepare the cut area, the absorbent member is cut at two cut points along its length into three equal portions. Either one of two cut areas for each cut point is chosen as a cut area (two cut areas in total). When the superabsorbent polymer particle is not a perfect sphere in those cut areas, an average of its minor axis and major axis is taken as a particle size of the particle. The term "backsheet side" as used herein is intended to include the portion of an absorbent member from the backsheet facing surface to half the thickness of the absorbent member. Accordingly, the term "particle size in the backsheet side" refers to the particle size as obtained in the same manner as for the particle size in the topsheet side.

To examine only the difference in size of the superabsorbent polymer particles, the topsheet facing surface of the absorbent member is observed with the naked eye instead of the above-described particle size measurement. To improve visibility of the superabsorbent polymer in the web 2, a 5% copper sulfate aqueous solution is sprinkled all over the absorbent member and, ten minutes later, excess water is wiped up with paper towel before observation.

The superabsorbent polymer embeddedly supported in the web 2 has a particle size distribution in the thickness direction of the web 2. As shown in Fig. 1, the superabsorbent polymer 3 has different particle sizes between the topsheet side and the backsheet side of the web2. In detail, the superabsorbent polymer 3 includes a polymer with a relatively larger particle size (hereinafter "larger size polymer") 3a and a polymer with a relatively smaller particle size (hereinafter "smaller size polymer") 3b. The larger size polymer 3a is localized in the topsheet side of the web 2, while the smaller size polymer 3b in the backsheet side.

The variation in particle size of the superabsorbent polymer 3 in the thickness direction of the web 2 may be stepwise or continuous. When the variation is stepwise, not all the polymer particles in the topsheet side need to have the same size. This also applies to the polymer in the backsheet side. In short, it is only necessary that the polymer in the topsheet side has a larger average particle size than that in the backsheet side.

The larger size polymer 3a being localized in the topsheet side, and the smaller size polymer 3b being localized in the backsheet side, the absorbent member 1 has an increased rate of absorption for the following reason. In general, a superabsorbent polymer with a larger particle size tends to have a smaller surface area per unit weight and a smaller absorption rate (speed of absorption). Additionally, superabsorbent polymer particles having a large particle size, being difficult to pack closest, form a large number of large spaces between them. Therefore, liquid having passed through a topsheet and reached the absorbent member 1 runs directly downward the absorbent member 1 without being rapidly absorbed by the larger size polymer 3 a localized in the topsheet side. Moreover, the continuous fiber web 2 is capable of passing liquid rapidly because of its sparser structure containing large interfiber spaces compared with an airlaid fluff pulp layer that has been widely used as an absorbent member. As a result, liquid rapidly reaches the backsheet side where the smaller size polymer 3b is distributed. Since the smaller size polymer 3b has a larger surface area and a higher absorption rate, the liquid is quickly absorbed by the smaller size polymer 3b. Easy to pack closest, the smaller size polymer particles 3b form a great number of fine interstices therebetween and therefore exert high capability of space retention. The portion of the liquid that has not been absorbed by the smaller size polymer 3b is absorbed by the larger size polymer 3a localized in the topsheet side. The absorbent member according to the present embodiment has an increased rate of liquid absorption based on the above-described absorption mechanism. Moreover, liquid swiftly migrates through the thickness of the absorbent member 1, and the portion of the liquid that may return toward the topsheet is absorbed by the larger size polymer 3a, little remaining between the larger size polymer particles 3a. As a result, the absorbent member 1 allows little liquid to remain in its topsheet side and to pass back through and thus keeps a feeling of dryness. Besides, since the spaces formed between the larger size polymer particles 3a are wider than those formed between the smaller size polymer particles 3b, swell (volume increase) of the larger size polymer 3a resulting from absorption can be absorbed by the spaces. As a result, gel blocking hardly occurs.

Even if superabsorbent polymer particles in an airlaid fluff pulp layer that has been widely used as an absorbent member have the same particle size distribution as in the present embodiment, an improvement in absorption rate cannot be expected because fluff pulp has an extremely lower rate of liquid permeation than the continuous fiber web and also because pulp collapses when wetted.

From all these considerations, the average particle size of the larger size polymer 3a localized in the topsheet side is preferably 300 to 700 $\mu$m, more preferably 350 to 500 $\mu$m, while that of the smaller size polymer 3b localized in the backsheet side is preferably 50 to 300 $\mu$m, more preferably 100 to 250 $\mu$m. The particle size of the superabsorbent polymer is measured by the method described above. The difference in superabsorbent polymer particle size between the topsheet and the backsheet sides of the web 2 is confirmed by, for example, observing cut areas of the web 2 under microscopic magnification. It is also confirmed by pouring water or physiological saline into the web 2 to swell the superabsorbent polymer and observing the swollen polymer particles with the naked eye. The size of the swollen superabsorbent polymer particles is proportional to the size before swelling.

In order to prevent the gel blocking more effectively, it is preferred for the superabsorbent polymer 3 to have high gel strength. A rate of liquid permeation under a load of 0.6 kPa is one of the measures representing gel strength of a superabsorbent polymer. Gel blocking and resultant reduction of absorbing performance can be prevented by using a superabsorbent polymer 3 having a rate of liquid permeation of 150 to 1500 g/min, more preferably 300 to 750 g/min. Using such a superabsorbent polymer 3 is also preferred for preventing leakage attributed to too slow absorption.

The rate of liquid permeation under load is measured as follows. A superabsorbent polymer weighing 0.5 g is put in 100 g of physiological saline to swell to saturation. The swollen superabsorbent polymer and the physiological saline are transferred into a cylinder having a cross-sectional area of 4.91 cm$^2$ (inner diameter: 25 mm) with its bottom closed with a cock (inner diameter: 4 mm) which can be freely opened and closed. After the swollen polymer sinks to the bottom, a glass filter (inner diameter: 23 mm, pore size: 160 to 250 $\mu$m; available from Shibata Scientific Technology, Ltd.) is put on the superabsorbent polymer, and a large number of glass beads having a diameter of 1 mm are placed thereon to apply a load of 0.6 kPa to the superabsorbent polymer. The cock is opened to let 50 ml of the physiological saline pass through. The time required for 50 ml of the saline to pass through is taken as a liquid permeation rate. The liquid permeation rate is one of measures reflecting gel strength of the superabsorbent polymer. The shorter the liquid permeation rate, the higher the gel strength.

The superabsorbent polymer 3 is uniformly embeddedly supported in the web 2 throughout the thickness of the web 2. The expression "embeddedly supported" as used herein is intended to indicate the state in which the superabsorbent polymer enters the interfiber spaces formed by the continuous fibers, particularly crimped continuous fibers, and therefore hardly moves extremely or falls off with the wearer's active movement. This state is the results of the continuous fibers' entangling with or catching on the superabsorbent polymer or the polymer's adhering to the continuous fibers by its own stickiness. The spaces among the continuous fibers are protected from destruction because the spaces are easily deformable when externally stressed and also because the stress is absorbed by the whole continuous fibers. The term "uniform" or "uniformly" is used not only when the superabsorbent polymer is distributed completely uniformly in the thickness or width direction of the absorbent member 1 but also when the variation in weight of the polymer per unit area among different parts of the absorbent member 1 is such that the maximum is within double the minimum. Such variation is attributed to a phenomenon infrequently faced in the manufacture of an absorbent article that the superabsorbent polymer is fed in excess and spread in a part in an extremely large amount. That is, under the term "uniform" is included an unavoidable nonuniform distribution, but an intentional variation in amount of the superabsorbent polymer is not included under the term.

In cases where the continuous fibers forming the web2 have crimp, they furnish a great number of spaces capable of retaining particles, in which spaces the superabsorbent polymer is held. Therefore, the superabsorbent polymer even if distributed in a large quantity hardly moves extremely in the web or falls off the web. Furthermore the structure of the absorbent member 1 is hardly destroyed by active movement of a wearer. The percentage of crimp and the amount of the continuous fibers are selected appropriately depending on the kind of the superabsorbent polymer used. A conventional absorbent member could hold a large quantity of a superabsorbent polymer by increasing the amount of a fibrous material, which results in increases in weight and thickness of the absorbent member. In contrast, the present invention makes it possible without difficulty to increase the amount of the superabsorbent polymer relative to the amount of the fibrous material. Specifically, the absorbent member 1 as a whole preferably contains the superabsorbent polymer in an amount equal to or larger than, more preferably twice or more as much as, even more preferably three times or more as much as, the weight of the continuous fibers. Such high polymer supporting properties has accomplished reduction in thickness and weight of the absorbent member 1. The maximum weight per unit area of the superabsorbent polymer supportable by the continuous fibers is decided in connection with prevention of extreme movement or fall-off of the polymer. While depending on the degree of crimping of the continuous fibers, the superabsorbent polymer hardly moves extremely or falls off against active wearer's movement as long as the weight of the superabsorbent polymer is not more than about ten times the weight of the continuous fibers.

The degree of the superabsorbent polymer being embeddedly supported can be evaluated by the following method. A web measuring 100 mm by 200 mm is cut along its lateral centerline to prepare a 100 mm x 100 mm test piece. The test piece is hang with a cut end downward and given 20 transverse shakes with an amplitude of 5 cm at a rate of one shake per second. The polymer fallen from the cut end is weighed. The proportion of the weight of the fallen polymer to the weight of the superabsorbent polymer initially contained in the cut piece before the test is obtained. When the thus calculated proportion is 25% by weight or less, preferably 20% by weight or less, more preferably 10% by weight or less (to put it in another way, when the proportion of the weight of the superabsorbent polymer remaining in the piece after the test to the weight of the superabsorbent polymer initially present in the piece (this proportion is called "supporting ratio") is 75% or more, preferably 80% or more, more preferably 90% or more), it is safe to say that the superabsorbent polymer hardly moves extremely or falls off.

The following method of evaluation using the test piece that has been subjected to the above-described supporting ratio measurement is also useful. Fifty grams of physiological saline (0.9 wt% NaCl) is evenly sprayed onto the piece that has been subjected to the supporting ratio measurement to observe the swell with the naked eye. When the variation in thickness of the swollen piece is such that the maximum is not larger than double the minimum, it is safe to say that the polymer hardly moves extremely or falls off.

The test piece to be used in the above-described methods of evaluation should be cut out of a region where the superabsorbent polymer is evenly distributed in the planar direction of the web.

The superabsorbent polymer 3 that can be used is selected from those commonly employed in conventional absorbent articles with no particular restriction. Exemplary superabsorbent polymers include sodium polyacrylate, acrylic acid-vinyl alcohol copolymers, crosslinked sodium polyacrylate, starch-acrylic acid graft copolymers, isobutylene-maleic anhydride copolymers and saponification products thereof, potassium polyacrylate, and cesium polyacrylate. In order for the superabsorbent polymer to satisfy the requirement with respect to liquid permeation rate under load, a crosslinking density gradient is provided on the surface of the superabsorbent polymer particles, or aspherical, irregularly shaped superabsorbent polymer particles are used. Specifically, the methods disclosed in U.S. Patent 5,865,822, col. 10,1. 62 to col. 12,1. 40 can be used, the disclosures of which are herein incorporated by reference. The amount of the superabsorbent polymer 3 in the web 2 is preferably 50 to 1000 $g/m^2$, more preferably 100 to 500 $g/m^2$.

The web 2 may contain organic or inorganic particles other than the superabsorbent polymer 3, including activated carbon, silica, alumina, titanium oxide, and various clay minerals (e.g., zeolite, sepiolite, bentonite, and cancrinite), as deodorants or antimicrobials. The inorganic particles may have part of their metal sites displaced. Various organic or inorganic buffers can also be used, including acetic acid, phosphoric acid, citric acid, succinic acid, adipic acid, malic acid, lactic acid, and salts of these acids, either individually or as a mixture thereof. Various amino acids are also usable. The function of these ingredients is to suppress the smell of the fluids absorbed by the absorbent member and the smell of the constituent materials *per se.* The organic or inorganic buffers also have a function of neutralizing ammonia generated by decomposition of excrement, e.g., urine, to maintain the diaper neutral to weakly acidic, which reduces the influences on the skin even if rewet occurs. Furthermore, the organic or inorganic buffers having a function of neutralizing an alkali such as ammonia are expected to protect such continuous fibers which are forming the web2 and having an intermolecular ester bond (e.g., cellulose acetate fibers) against damage due to ester bond cleavage by an alkali.

The web may further contain hydrophilic fine powder or staple fiber to improve the liquid retention, rate of absorption, and dryness. Examples of the hydrophilic fine powder or staple fiber include fibrillated or non-fibrillated cellulose powder, carboxymethyl cellulose and metal salts thereof, carboxyethyl cellulose and metal salts thereof, hydroxyethyl cellulose and derivatives thereof, silk powder, nylon powder, and rayon, cotton or wool staple fibers. Preferred of them is cellulose powder for being the most effective of the others. The hydrophilic fine powder or staple fiber may be distributed on the web before the superabsorbent polymer is distributed or be previously mixed with the superabsorbent polymer to be spread on the web.

The web 2 in which the superabsorbent polymer 3 is embeddedly supported is made up of continuous fibers having hydrophilic properties. The continuous fibers having hydrophilic properties that can be used in the present invention include those which are essentially hydrophilic and those which are not essentially hydrophilic but rendered hydrophilic through a hydrophilization treatment. Essentially hydrophilic continuous fibers are preferred. Continuous fibers of nylon, acrylic resins, cellulose acetate or rayon are more preferred. Cellulose acetate continuous fibers with a water content of less than 10% are particularly preferred for bulk retention even when wetted. Cellulose acetate is preferably cellulose triacetate or cellulose diacetate. The water content as referred to above is a value measured in an environment at 25°C and 65% RH.

The continuous fibers are preferably crimped fibers. The crimped continuous fibers preferably have a crimp percentage (specified in JIS L0208) of 10% to 90%, more preferably 10% to 60%, even more preferably 10% to 50%. By making a web from crimped continuous fibers, it is easy to embeddedly support a large quantity of a superabsorbent polymer in the web stably, and the superabsorbent polymer, even when used in a large amount, is suppressed from moving extremely or falling off. For example, the relationship between crimp percentage of the continuous fibers and superabsorbent polymer supporting ratio in the web is as shown in Table 1 below. It is seen from Table 1 that very high superabsorbent polymer-supporting ratios can be achieved when the crimp percentage of the continuous fiber falls within a specific range. The continuous fibers in Table 1 had a thickness of 2.1 dtex. A tow of the continuous fibers was conveyed under tension and opened or spread out into a web using an air opening apparatus. The tow-opened web was then combed between a roll having a number of discs spacedly arranged around its periphery along the axial direction and a smooth backup roll. The width of the web was adjusted to 100 mm, the running speed of the web was slowed down, and the web was transferred onto a vacuum conveyor. The tension given to the web on the vacuum conveyor was relaxed to a controlled degree to develop crimps. In this way, webs of cellulose acetate continuous fibers having various crimp percentages were prepared according to the degree of relaxation. Relaxing the web from tension by above methods widened the interfiber spaces to help a superabsorbent polymer to enter the web and increased the web thickness to improve the superabsorbent polymer supporting capacity. A superabsorbent polymer was then spread on the web and embeddedly supported in the tow-opened web. The weight of the web was 26 g/m$^2$. The amount of the polymer spread was 260 g/m$^2$. Lumpy polymer particles with an average particle size of 330 $\mu$m were used. The resulting absorbent members were tested for structural stability while dry in accordance with the test method described later. The weight of the superabsorbent polymer remaining after the test was divided by the weight of the superabsorbent polymer initially contained in the web, and the quotient was multiplied by 100 to give a superabsorbent polymer supporting ratio (%). The values in Table 1 are to vary depending on the thickness and amount of the continuous fibers used, the mixing ratio of the superabsorbent polymer, the shape and hygroscopicity of the superabsorbent polymer, and the like.

Table 1

| Crimp Percentage of Continuous Fiber in Web (%) | Superabsorbent Polymer Supporting Ratio (%) |
| --- | --- |
| 0 | 0 |
| 14 | 80.5 |
| 20 | 86 |
| 24.5 | 97.5 |
| 29.8 | 95.2 |
| 62 | 67.5 |

The means for crimping the continuous fibers is not particularly limited. The crimp may be either two-dimensional or three-dimensional. The percentage of crimp (or crimp percentage) is defined to be a percentage of a difference between the length A of a continuous fiber in its straightened state and the natural length B of the continuous fiber to the length A, being calculated from equation:

$$\text{Percentage of crimp (\%)} = ((A-B)/A) \times 100$$

The natural length of a continuous fiber is the length of the straight line connecting the two ends of a continuous fiber in its natural state. The term "natural state" means a state of a continuous fiber hanging under its own weight with its one end fixed to a horizontal plate. The term "the length of a continuous fiber in its straightened state" means the length of a continuous fiber stretched out until no crimp remains under a minimum load.

The number of crimps of the continuous fibers having the recited percentage of crimp is preferably 2 to 25, more preferably 4 to 20, even more preferably 10 to 20, per centimeter.

The thickness of the continuous fibers is not particularly limited. Satisfactory results can generally be obtained by using continuous fibers having a thickness of 1.0 to 7.8 dtex, preferably 1.7 to 5.6 dtex. The terminology "continuous fiber" as used throughout the description means a fiber having a fiber length preferably of 70 mm or longer, more preferably 80 mm or longer, even more preferably 100 mm or longer, as measured by the mean fiber length measurement method (method C) specified in JIS L1015. In cases where the length of a web *per se* is shorter than 100 mm, "continuous fiber" is defined as follows. When preferably at least 50%, more preferably 70% or more, even more preferably 80% or more, of the fibers making up the web extend over the whole length of the web, the fibers of the web are regarded as continuous fibers. The continuous fibers used in the present invention are those generally termed "continuous filaments". A bundle of continuous filaments unidirectionally oriented is generally termed "a tow". Accordingly, the terminology "continuous fiber" as used herein shall include a continuous filament. The expression "a web having continuous fibers oriented" or its equivalent shall include a bundle of continuous fibers, namely, a tow, as a raw material for making a web and a tow layer of continuous filaments. It is acceptable that the absorbent member contains fibers whose length is shorter than the above recited length as a result of the continuous fiber being cut.

In the absorbent member 1, the continuous fibers making up the web 2 are unidirectionally oriented in the planar direction of the absorbent member 1. Owing to the unidirectional fiber orientation, liquid absorbed by the absorbent member 1 diffuses preferentially in the orientation direction of the continuous fibers, i.e., in the planar direction of the absorbent member 1. In contrast, liquid diffusion in the direction perpendicular to the orientation direction of the continuous fibers is suppressed. When the continuous fibers are oriented in the length direction of the absorbent article, side leakage (leakage from both sides of the absorbent article) is effectively prevented.

It is only necessary that the continuous fibers are oriented such that the vector connecting the leading end and the trailing end of the individual fiber is in the planar direction. It is acceptable that part of the individual continuous fibers is vertically directed (in the thickness direction of the absorbent member) due to twisting or entanglement between the leading and trailing ends. The degree of orientation of the continuous fibers is preferably 1.2 or higher, more preferably 1.4 or higher, as measured with a microwave molecular orientation analyzer MOA-2001A manufactured by KANATA Co., Ltd. Measurement is made on three points of a sample measuring 50 mm in lateral direction and 100 mm in longitudinal direction to obtain an average. Where a sample is smaller than that size, two or more samples adjoining to each other without overlap are used.

Where the continuous fibers are oriented in the longitudinal direction of an absorbent article, it is preferred that there is no linear joint line across the orientation direction of the continuous fibers in the absorbent member. Such a joint line would block smooth fluid distribution in the orientation direction, which can cause side leaks.

Where the continuous fibers are oriented in the lateral direction of an absorbent article, downward wicking properties are obtained with longitudinal fluid distribution suppressed. In this case, it is preferred that the absorbent member has a joint line across the orientation direction of the continuous fibers to prevent side leakage. The term "line" in "joint line" means a continuous line capable of inhibiting penetration of a liquid. This does not mean that the individual sealing lines, etc. must be linked to one another continuously. For instance, when sealing lines formed spacedly but with overlaps when seen from a certain direction are capable of hindering migration of a liquid in that direction, the sealing lines can be said to be a joint line. The joint line may be curved or piecewise straight as well as straight linear. The joint line width is preferably about 0.2 to 15 mm.

A joint line may be formed only in the web 2, or a linear joint may be such that the topsheet is joined to the absorbent member. In any configuration, a joint line is preferably formed in the longitudinally middle portion of the absorbent article. A joint line may be formed outboard of both lateral side edges of the absorbent member. If a liquid moves in the web through capillarity, it will run into the joint line and is blocked from moving farther by the above-mentioned configuration of a joint line, thereby prevented from causing side leakage.

A preferred process of producing the absorbent member 1 will be described. First of all, a tow of continuous fibers is prepared. The tow is conveyed while being stretched under tension in the machine direction. In this state, the tow is opened into a web. The step of opening the tow is achieved using, e.g., an air opening apparatus utilizing compressed air. In the case of crimped continuous fibers, the continuous fibers easily stretch under tension in the machine direction. A superabsorbent polymer is spread on the stretched web. Prior to polymer spreading, the running speed of the web is slowed down, and the web is transferred onto a vacuum conveyor. While on the vacuum conveyor, the tension given to the web is relaxed to release the web from stretch. In the case of crimped continuous fibers, the continuous fibers return to their crimped state on release from stretch. In addition, the web becomes bulkier than it has been under tension. As a result, the web gains improved ability to hold a superabsorbent polymer. In that state, a superabsorbent polymer is spread. The crimped continuous fibers have interfiber spaces in which the superabsorbent polymer can be embedded and retained. In the interfiber spaces, the superabsorbent polymer is embeddedly supported. The web is sucked from the reverse side simultaneously with the polymer spreading to help the polymer be embeddedly supported. It is important to feed the superabsorbent polymer to the tow-opened web while the web is under a relaxed tension.

The fibers constituting the web preferably have an average interfiber distance within plus/minus one sigma of the average particle size of the superabsorbent polymer, more preferably plus/minus 0.5 sigma, wherein sigma is the standard deviation of the average particle size. The average interfiber distance of the web is calculated from equation:

[equatim1]

$$\mathbf{D_p} = \frac{1.485 \times 10^4}{\sqrt{\mathbf{W}/\mathbf{F}}} - \mathbf{fd}$$

where $D_p$ is an interfiber distance ($\mu$m); F is a fineness (denier), W is a packing density (g/m3) of fibers; and fd is a diameter ($\mu$m) of fibers.

In the calculation of average interfiber distance, the web constituting fibers are all regarded to have a circular cross-section. In the case of a modified cross-section, an average of the major axis and the minor axis is taken as a diameter. In the case of a Y-shaped or C-shaped cross-section, the circumscribed circle diameter is taken.

The superabsorbent polymer can be spread using a hooded feeder combined with the vacuum conveyor. Air in the hood is sucked by the vacuum conveyor. The superabsorbent polymer particles fed from the feeder are classified while being carried along the air stream; for larger particles receive greater air force and are carried farther in the air stream. The superabsorbent polymer particles thus classified are embedded and supported in the web by the suction of the vacuum conveyor successively in the order from small to large sizes.

In general, the superabsorbent polymer 3 is not an assembly of particles of a size but an assembly of particles of various sizes and has a particle size distribution such as a normal distribution. Accordingly, when the superabsorbent polymer 3 is spread on the tow-opened web, the tow-opened web serves like a sieve to capture large superabsorbent polymer particles 3 in the vicinity of the upper surface and to allow smaller superabsorbent polymer particles 3 to enter deeper into the web. The fact that the web is sucked on its side opposite to the side where the polymer particles 3 are fed and the fact that the particles 3 are classified in the air stream help the smaller particles enter deeper toward the backsheet side of the web. In this way, the superabsorbent polymer 3 is distributed and embeddedly supported in the web with its particle size gradually decreasing from the side on which the polymer 3 is spread to the opposite side.

After the superabsorbent polymer is spread on the web, the web is wrapped in tissue paper or nonwoven fabric of various kinds having an adhesive, such as a hot-melt adhesive, applied thereto by a contact application method, such as roll coating or screen printing, or a non-contact application method, such as spray coating. Spray coating, a non-contact application method, is preferred for easy changeover of application patterns and adjustability of the amount of the adhesive to be applied. In particular, a spray coating system fit for bonding in points is preferred. Spray coating systems include slot spraying, curtain spraying, melt blown spraying, and spiral spraying. The amount of the adhesive to be applied is preferably as small as not to become a hindrance to migration of liquid. From this viewpoint, the amount of the adhesive to be applied preferably ranges from 3 to 30 g/m2, more preferably 5 to 15 g/m2.

The web may be compressed by means of a roll, a belt, etc. to ensure adhesion between the superabsorbent polymer 3 and the continuous fibers and to embed the superabsorbent polymer 3 in the web more markedly. The superabsorbent polymer is thus embeddedly supported in the web 2 with a particle size distribution in the thickness direction of the web.

Taking advantage of the function of the web 2 to sieve the superabsorbent polymer particles, the web 2 may have higher fiber content regions Fa and lower fiber content regions Fb both extending along the orientation direction of the continuous fibers. The regions Fa and Fb alternate in the direction perpendicular to the orientation direction of the continuous fibers. The higher fiber content region Fa is a region having a relatively higher fiber content per unit area of the web, and the lower fiber content region Fb is a region having a relatively lower fiber content per unit area of the web, both on a cross-section along the web's thickness direction.

The higher fiber content region Fa is less easy for the superabsorbent polymer to pass through in the thickness direction as compared with the lower fiber content region Fb. This makes selective distribution of the superabsorbent polymer by sieving more complete. As remarked, the smaller size superabsorbent polymer particles do not need to be distributed uniformly in the backsheet side and may be localized in part of the backsheet side.

The width of each of the higher fiber content region Fa and the lower fiber content region Fb (the dimension of the regions in the direction perpendicular to the orientation direction of the continuous fibers) is not critical in the present embodiment. The widths of the two types of regions may be the same or different. Where different, the higher fiber content region Fa may be wider than the lower fiber content region Fb, or otherwise. In an extreme case which depends on the method of making the web 2, the width of the lower fiber content region Fb is far smaller than that of the higher fiber content region Fa.

While depending on the method of making the web 2, the width of each of the high fiber content region Fa and lower fiber content region Fb is preferably 0.5 to 20 mm, more preferably 2 to 10 mm, for clear visibility of the stripe pattern through the topsheet and for the improvement of absorptivity. The stripe pattern has an advantage of adding visual dryness to a wearer.

The fiber content change between the higher fiber content region Fa and the lower fiber content region Fb may be either stepwise or continuous. The fiber content in the higher fiber content region Fa is preferably 10 to 200 $g/m^2$, more preferably 20 to 100 $g/m^2$, and that in the lower fiber content region Fb is preferably 20 $g/m^2$ or less, more preferably 3 to 10 $g/m^2$. The fiber content W1 in the higher fiber content region Fa is higher than the fiber content W2 in the lower fiber content region Fb. Preferably the fiber content W1 in the higher fiber content region Fa is double or more than double the fiber content W2 in the lower fiber content region Fb.

In another method for nonuniformly distributing the smaller size superabsorbent polymer particles in the backsheet side by making use of the sieving effect of the web, the weight per unit area, the constituent fibers, or the density of the web is varied. For example, a thicker region may be formed by superposing on the web another tow-opened web having a different spread width. In this method, the thicker region exhibits enhanced sieving effect on the superabsorbent polymer particles, resulting in the formation of a nonuniform distribution of the smaller size particles in the backsheet side. The thicker region is preferably formed in the laterally middle portion of the web. By so doing, the sieving effect on the superabsorbent polymer particles is manifested in the laterally middle portion. As a result, the larger size superabsorbent polymer particles allow liquid to pass through quickly in the skin side, whilst the smaller size superabsorbent polymer particles in the backsheet side rapidly immobilize the liquid. Since the superabsorbent polymers in the peripheral portion of the absorbent member are present without being sieved, liquid penetration in the peripheral portion is delayed compared with the middle portion. This is effective to suppress leakage.

In still another method, two or more webs made of fibers having different thicknesses are superposed on one on top of another. The skin side is formed of a web of thicker fibers, while the backsheet side is formed of a web of finer fibers. In this case, too, the webs to be superposed may have different widths. The upper web of thicker fibers has a longer average interfiber distance so that only still larger size particles remain thereon. Accordingly, a combined use of the webs of fibers of different thicknesses provides a particle size distribution in the thickness direction. The same effects can be obtained by using upper and lower webs having different crimp percentages.

The above-described methods may be replaced with a method in which part of the web is previously compressed to be densified and therefore to have a reduced sieving effect. Conversely, spreading the superabsorbent polymer may be followed by compressing part of the web to promote embedding the superabsorbent polymer inside the web.

A superabsorbent polymer-free web may be superposed on the above-described web having a particle size distribution in its thickness direction. Both edge portions of a web where the superabsorbent polymer is not spread may be folded over the upper or lower side of the web to provide a superabsorbent polymer-free layer on both edge portions. In other words, the present invention includes in its scope a configuration in which a superabsorbent polymer-free web layer is present as an outermost layer on the skin side or the backsheet side.

The amount of the fibers in a web is measured by weighing a piece measuring 100 cm x 100 cm cut out of the web and calculating the weight per unit area when the fibers are distributed over a sufficiently broad area. On the other hand, when it is difficult to cut out a piece of a certain size sufficient to weigh, for example, when the fibers are distributed in a stripe pattern, the following method can be taken. A load of 24.5 kPa is applied to an absorbent member to be measured for 12 hours beforehand to prepare a sample freed from the influences such as thickness recovery and wrinkles. First of all, a cut piece measuring 10 cm by 10 cm is weighed to calculate the weight per unit area of the whole absorbent member. The amount of the superabsorbent polymer is then determined. The weight per unit area of the superabsorbent polymer is subtracted from the weight per unit of the absorbent member to obtain an average weight per unit area of the fibers in the absorbent member. The amount of the superabsorbent polymer in the absorbent member is determined by measuring the overall weight and area of the absorbent member, immersing the absorbent member in an ascorbic acid solution, followed by exposure to sunlight, to dissolve the superabsorbent polymer, washing away the dissolved polymer with water, and weighing residual fibers. Then, the absorbent member is cut along a direction perpendicular to the continuous fiber orientation direction. A cut area corresponding to a width of 50 mm is dividedly scanned into a computer as image data. Each picture, taken of an area of 5 mm by 5 mm, is enlarged 25 times and inputted in sequence. The images are processed with image processing software (Image-Pro Plus, from Media Cybernetics) to calculate the area occupied by the fibers (the sum of the cross-sectional areas of the individual fibers) per image. The measurement was taken on five points to obtain an average. Assuming that the web has a uniform thickness, the area occupied by the fibers (referred to as an average area) is previously calculated from previously determined average weight per unit area of the fibers and the cross-sectional area of a single fiber. A region in which the fibers occupy an area equal to or more than the average area is regarded as a higher fiber content region, and a region in which the fibers occupy an area less than the average area is regarded as a lower fiber content region.

According to the aforementioned method, superabsorbent polymer particles can be embeddedly supported in the web 2 with a particle size distribution in the thickness direction of the web easily by using only one kind of a polymer. The same configuration could be obtained by a different method using two or more kinds of polymers different in particle size distribution, but such involves complicated steps and encounters difficulty in increasing production efficiency. Furthermore, using two or more kinds of polymers gives rise to another problem that the polymers in a mixed state will exhibit complicated absorption characteristics attributed to the difference between them in absorption characteristics, making it difficult to obtain an absorbent member with desired absorption characteristics.

The thus obtained absorbent member 1 is thin and yet has high absorption capacity. In application to disposable diapers for babies, for example, the absorbent member 1 is preferably thin, having a thickness of 1 to 4 mm, more preferably 1.5 to 3 mm. In application to sanitary napkins, the thickness is preferably 0.5 to 3 mm, more preferably 1 to 2 mm. For applications to incontinence pads, the thickness is preferably 0.5 to 4 mm, more preferably 1 to 3 mm. Absorbent articles having the absorbent member 1 with a thickness in the recited ranges will be of thin type preferably having a thickness of 1 to 10 mm. The thicknesses of the absorbent member 1 and the absorbent article are measured with a load of 0.245 kPa applied thereto by placing a 5 cm square acrylic resin plate and a weight. In the present embodiment, the thickness was measured with a laser displacement sensor LK080 class 2 available from Keyence Corp. Measurement is made at five positions to obtain an average. If a piece of data fluctuates 20% or more, the piece is tossed out and replaced with an additional one. Prior to the measurement, a load of 24.5 kPa is applied to the sample for 12 hours to straighten wrinkles.

To obtain the above-recited thicknesses with ease, it is preferred for the absorbent member 1 to have a weight of 120 to 400 $g/m^2$, more preferably 150 to 300 $g/m^2$, for applications to disposable diapers for babies; 35 to 200 $g/m^2$, more preferably 50 to 150 $g/m^2$, for applications to sanitary napkins, or 35 to 500 $g/m^2$, more preferably 50 to 400 $g/m^2$, for applications to incontinence pads.

Figs. 2 to 4 illustrate a flat type disposable diaper as an example of the absorbent article having the absorbent member according to the present embodiment. A diaper 10 is characterized in that a pair of barrier cuffs extending in the longitudinal direction of the diaper 10 are doubly provided. The diaper 10 has a topsheet 11 and a backsheet 12. The absorbent member 1 is interposed between the two sheets 11 and 12. On the exterior side of the backsheet 12 is provided a water repellent exterior sheet 13 formed of nonwoven fabric. A leg flap 20 extends laterally outward from both side edges of the absorbent member 1.

Elastic threads 21 extending in the longitudinal direction of the diaper 10 are disposed in their stretched state in the side edge portion of each leg flap 20 to form leg barrier cuffs 22. Between each leg barrier cuff 22 and each side edge of the absorbent member 1 are provided a first barrier cuff 23 and a second barrier cuff 24, both rooted in the leg flap 20. That is, two pairs of opposing barrier cuffs are provided. The first barrier cuff 23 is nearer to the leg barrier cuff, and the second barrier cuff 24 to the absorbent member. Each of the barrier cuffs 23 and 24 extends in the longitudinal direction of the diaper 10. An elastic thread 25 is disposed in its stretched state along the free edge of each of the barrier cuffs 23 and 24. Each of the barrier cuffs 23 and 24 is formed of a single sheet of, for example, water repellent nonwoven fabric.

The base of the second barrier cuff 24 is bonded to the topsheet 11. The base of the first barrier cuff 23 is bonded to the backsheet 12. The elastic thread 21 in the leg barrier cuff 22 is sandwiched between a lateral extension from the base of the first barrier cuff 23 and the exterior sheet 13.

The three barrier cuffs located in each leg flap 22 are preferably designed such that the outermost one may have a higher contractibility than the rest of three. That is, the contractive forces of the leg barrier cuff 22, the first barrier cuff 23, and the second barrier cuff 24 being taken as L1, L2, and L3, respectively, a preferred relation is L1>L2 and L1>L3. It is more preferred that the contractive force gradually decreases from the outermost to the innermost, i.e., L1>L2>L3 for the following reasons.

Absorbent articles have been designed based on the concept that thickness reduction without causing leakage could be accomplished by providing barrier cuffs having high contractibility so as not to leave a gap between a wearer's body and the absorbent article. However, strongly contractible barrier cuffs tend to leave marks on the skin and, when combined with a thin and flexible absorbent member as provided in the present embodiment, tend to curl up the absorbent article, making it difficult to put the absorbent article on a wearer. Moreover, too strong contractive force of barrier cuffs creates downward force to cause displacement while worn. These inconveniences associated with conventional absorbent articles can be averted by providing a pair of leg barrier cuffs and two or more pairs of opposing barrier cuffs with their contractibility satisfying the above-described relation.

The contractive force of a barrier cuff is measured as follows. A barrier cuff is cut out from an absorbent article to obtain a specimen. The specimen is tested on a tensilon tester ORIENTEC RTC-1150A to plot a hysteresis curve. The stress in the retracting curve is taken as a contractive force. The pulling and retracting speeds are 300 mm/min.

The initial span length of the specimen is 100 mm, and the maximum elongation is 100 mm (stretched to double the initial length). The stress at 50 mm back from the maximum stretched length in the retracting curve of the hysteresis is read as a contractive force of the specimen. Measurement is made on five specimens per sample to obtain an average. When the maximum elongation does not reach 100 mm, the stress required for stretching the specimen to an elongation of 50 mm is taken as a contractive force of the specimen.

The contractibility of the barrier cuffs can be adjusted by, for example, changing at least one of the thickness, extensibility, and the number of the elastic members.

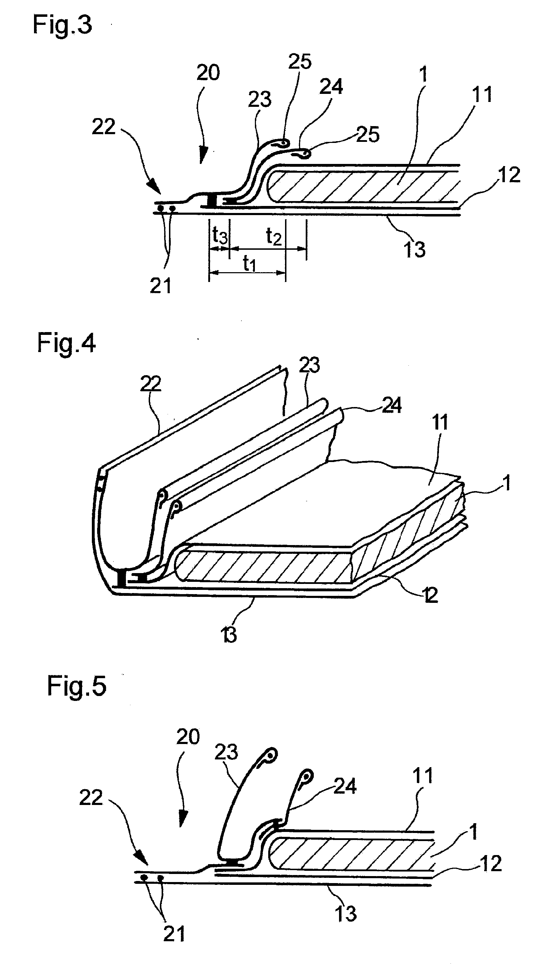

Since the barrier cuffs of the diaper 10 have so controlled contractive forces, they stand up to different heights according to their contractive forces while the diaper 10 is worn (or in its relaxed state) as shown in Fig. 4. The leg barrier cuff 22 having the highest contractive force stands highest, and the second barrier cuff 24 having the lowest contractive force stands lowest. The height of the first barrier cuff 23, the contractive force of which is intermediate between the leg barrier cuff 22 and the second barrier cuff 24, is intermediate between the heights of the leg barrier cuff 22 and the second barrier cuff 24. In brief, the higher the contractive force of the barrier cuff, the higher the height in the standing position. This is because a barrier cuff with a higher contractive force contracts more while the diaper 10 is worn (or in its relaxed state), and the larger the reduction in area due to the contraction, the more upright the barrier cuff rises.

In order for the barrier cuffs to show increasing standing heights from the inboard side to the outboard side of the leg flap 20, it is preferred that the length t1 of the first barrier cuff 23 from its base to its free edge is larger than the length t2 of the second barrier cuff 24 from its base to its free edge. So designed, the second barrier cuff 24 is prevented from hovering above the topsheet 11.

It is also preferred that the length t1 of the first barrier cuff 23, from its base to its free edge, is larger than the distance t3 between the base of the first barrier cuff 23 and the base of the second barrier cuff 24. The first and second barrier cuffs 23 and 24 so designed provide an adequate space between them. A liquid having run off the edge of the second barrier cuff 24 can be pooled in that space and prevented from further spilling over the first barrier cuff 23. The space also helps both the first and second barrier cuffs 23 and 24 to fit to the wearer's body while worn. While in the present embodiment the first and second barrier cuffs 23 and 24 are rooted at different positions, they may be rooted at the same position to rise up from the same position.

As shown in Fig. 2, the elastic threads 25 in the barrier cuffs 23 and 24 extend over the whole length of the diaper 10 so that the barrier cuffs 23 and 24 are contractive over the whole length of the diaper 10. On the other hand, the elastic threads 21 of the leg barrier cuffs 22 are disposed only in the crotch portion so that the leg barrier cuffs 22 are contractive only in the crotch portion. If the elastic threads 21 of the leg barrier cuff 22 extend up to the front portion and/or the rear portion of the diaper, contraction occurs in the front portion and/or the rear portion, which can impair operationality of fastening tapes 26 or make it difficult to attach the fastening tapes 26 to a target tape or landing tape (not shown).

While not shown in Figs. 2 to 4, a sheet independent of the topsheet 11 may be inserted between the topsheet 11 and the absorbent member 1. The independent sheet, herein called sublayer sheet, is provided for smoothing liquid passage from the topsheet 11 to the absorbent member 1 and suppressing rewet. The provision of the sublayer sheet also contemplates suppressing deterioration of the hand due to the larger size superabsorbent polymer particles on the skin side of the web. For these purposes, the sublayer sheet is preferably formed of a bulky sheet with some thickness. The bulkiness of the sublayer sheet secures the space through which liquid is transferred from the topsheet 11 to the absorbent member 1 and which accelerates smooth liquid passage. From that viewpoint, the sublayer sheet is preferably formed of a hydrophilic nonwoven fabric or an aggregate of hydrophilic staple fiber and preferably has a thickness of 0.1 to 5 mm, more preferably 0.2 to 3 mm. Suitable nonwoven fabrics include air-through nonwoven fabric and airlaid nonwoven fabric for ease of obtaining high bulkiness. The hydrophilic staple fiber aggregate is preferably a bulky aggregate of fibers that are slightly less hydrophilic than natural pulp. Examples include HBA pulp (interfiber- or intrafiber-crosslinked high bulk pulp), curly cellulose pulp, cellulose triacetate staple, and cellulose diacetate staple, and mixtures thereof. Tissue paper or nonwoven fabric of various kinds laminated with such a staple fiber aggregate is also useful.

A synthetic fiber layer may be disposed underneath the web 2. The synthetic fiber layer is made of synthetic fibers fused together into the form of a sheet. The web 2 and the synthetic fiber layer are bonded to each other. In the cases where the synthetic resin making the synthetic fibers and the material of the continuous fibers making the web 2 are incompatible with each other, part of the continuous fibers making the web 2 go through or enter the synthetic fiber layer to produce engagement between the continuous fiber layer and the synthetic fiber layer, whereby the web 2 and the synthetic fiber layer are joined together. Where the synthetic resin of the synthetic fibers and the material of the continuous fibers constituting the web 2 are compatible with each other, the web 2 and the synthetic fiber layer 4 can be fusion bonded. Otherwise, the web 2 and the synthetic fiber layer may be joined together with a commonly employed pressure-sensitive adhesive such as a hot melt adhesive. Even the absorbent member 1 is deformed by the wearer's movement, the smaller size superabsorbent polymer 3 in the backsheet side of the web 2 is effectively prevented from falling off by the synthetic fiber layer joined to the lower side of the web 2. Furthermore, the presence of the synthetic fiber layer reduces the gel feeling inherent to the liquid-swollen superabsorbent polymer 3 that might be felt by a wearer.

The synthetic fiber layer has fine interfiber gaps to keep air permeability and yet not to allow the superabsorbent polymer particles to pass through. Therefore, the synthetic fiber layer causes no impairment to the air permeability of the web 2.

The synthetic fibers include those called staple fibers preferably with an average length of 0.1 to 80 mm, more preferably 0.5 to 60 mm, even more preferably 1.5 to 51 mm. The fineness of the synthetic fibers is preferably 0.1 to 7.8 dtex, more preferably 0.5 to 5.6 dtex, even more preferably 0.9 to 3.4 dtex. Examples of useful synthetic fibers include single-component fibers made of polyethylene, polypropylene, polyethylene terephthalate, etc. and composite fibers made of two or more of these resins. The composite fibers include sheath-core or side-by-side bicomponent fibers. The synthetic fibers may have a circular cross-section, a modified cross-section, a C-shaped cross-section, or a hollow cross-section and so on.

Synthetic pulp can be used as synthetic fiber. Exemplary synthetic pulps include those based on a thermoplastic resin such as polyethylene, modified polyethylene or polypropylene. It is preferred for the synthetic pulp to have an average fiber length of 0.1 to 10 mm, more preferably 0.5 to 5 mm, even more preferably 0.9 to 1.5 mm, so that it may be handled in the same way as for natural pulp such as wood pulp.

The synthetic fiber layer 4 preferably weighs 3 to 30 g/m$^2$, more preferably 5 to 20 g/m$^2$, even more preferably 8 to 15 g/m$^2$, to prevent fall-off of the superabsorbent polymer, to reduce a gel feeling of the swollen superabsorbent polymer, and to reduce skin trouble and discomfort during use. The synthetic fiber layer may be textures by, for example, embossing.

While in the embodiment shown in Figs. 2 to 4 the base of the first barrier cuff 23 and the base of the second barrier cuff 24 are both located in the leg flap 20, the base of the second barrier cuff 24 may be located on the absorbent member 1 as shown in Fig. 5. While not shown, it is preferred to make holes through the joint of the base of the second barrier cuff 24 and the topsheet 11 so that a liquid pooled between the first and second barrier cuffs 23 and 24 may be absorbed by the absorbent member 1 through the holes. In making such holes, it is advantageous to join the base of the second barrier cuff 24 and the topsheet 11 by ultrasonic welding to carry out joining and hole making simultaneously.

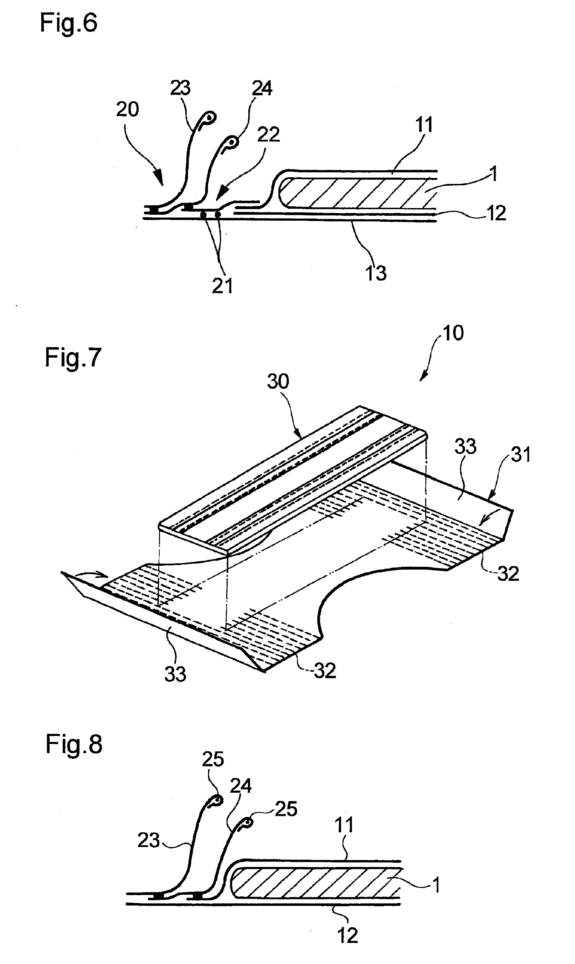

While in the embodiment shown in Figs. 2 to 4 the second barrier cuff 24, the first barrier cuff 23, and the leg barrier cuff 22 are arranged in that order from the inboard side to the outboard side of the leg flap 20, the three barrier cuffs may be arranged in the configuration shown in Fig. 6. In the embodiment shown in Fig. 6, the leg barrier cuff 22, the second barrier cuff 24, and the first barrier cuff 23 are arranged in that order from the inboard side to the outboard side of the leg flap 20. That is, the leg barrier cuff 22 in the embodiment of Fig. 6 is the most inboard. In contrast, the leg barrier cuff 22 in the embodiment of Figs. 2 to 4 is the most outboard. In the embodiment of Figs. 2 to 4 too, it is preferred that the contractive force gradually decreases from the most outboard to the most inboard barrier cuffs. That is, the contractive forces of the leg barrier cuff 22, the first barrier cuff 23, and the second barrier cuff 24 being taken as L1, L2, and L3, respectively, a preferred relation is L2>L3>L1 for the same reasons as mentioned with respect to the embodiment shown in Figs. 2 to 4.

Fig. 7 represents application of the absorbent member of the present embodiment to a pull-on type disposable diaper. Fig. 7 is an exploded perspective of a pull-on diaper in a flat-out state with its side seals opened. A diaper 10 includes an absorbent assembly 30 and an exterior sheet 31 disposed on the outer side of the absorbent assembly 30.

Fig. 8 is a fragmentary, transverse cross-section of the crotch portion of the absorbent assembly 30 shown in Fig. 7. The absorbent assembly 30 includes a topsheet 11 and a backsheet 12. The absorbent member 1 is interposed between the sheets 11 and 12. The exterior sheet 31 is joined to the outer side of the backsheet 12. While not shown, the aforementioned sublayer sheet may be inserted between the topsheet 11 and the absorbent member 1. The exterior sheet 31 is formed of two water-repellent sheets formed of nonwoven fabric having elastic members 32 fixed transversely therebetween in their stretched state. The elastic members 32 extend in the lateral direction of the diaper in non-intersecting relation to the absorbent assembly 30. The front and rear ends 33 of the exterior sheet 31 are each folded inward over the front and rear ends of the absorbent assembly 30.

The absorbent assembly 30 has a pair of first barrier cuffs 23 and a pair of second barrier cuffs 24 rooted outboard of each side edge of the absorbent member 1. That is, the absorbent assembly 30 has two pairs of opposing barrier cuffs. The absorbent assembly 30 does not have leg barrier cuffs. Also, the exterior sheet 31 does not have leg barrier cuffs.

In the absorbent assembly 30, the first barrier cuff 23 is laterally farther from the absorbent member, and the second barrier cuff 24 is laterally closer to the absorbent member. Both the barrier cuffs 23 and 24 extend in the longitudinal direction of the absorbent assembly 30. An elastic thread 25 is fixed in its stretched state along the free edge of each barrier cuff 23, 24. The base of the second barrier cuff 24 is bonded to the topsheet 11, and the base of the first barrier cuff 23 is bonded to a laterally outward extension from the base of the second barrier cuff 24 and to the backsheet 12.

In the present embodiment, the contractive force of the barrier cuffs are preferably designed such that the contractive force of the outboard first barrier cuff 23 is greater than that of the inboard second barrier cuff 24. That is, the contractive forces of the first barrier cuff 23 and the second barrier cuff 24 being taken as L2 and L3, respectively, a preferred relation is L2>L3 for the same reasons as mentioned with respect to the embodiment shown in Figs. 2 to 4.

While the diaper 10 is worn (or in a relaxed state), the standing height of the first barrier cuff 23 with a stronger contractive force is higher than that of the second barrier cuff 24 with a weaker contractive force. The reason therefor is the same as mentioned with respect to the embodiment shown in Figs. 2 to 4. To achieve such a configuration, it is preferred that the length from the base to the free edge of the first barrier cuff 23 is larger than that of the second barrier cuff 24.

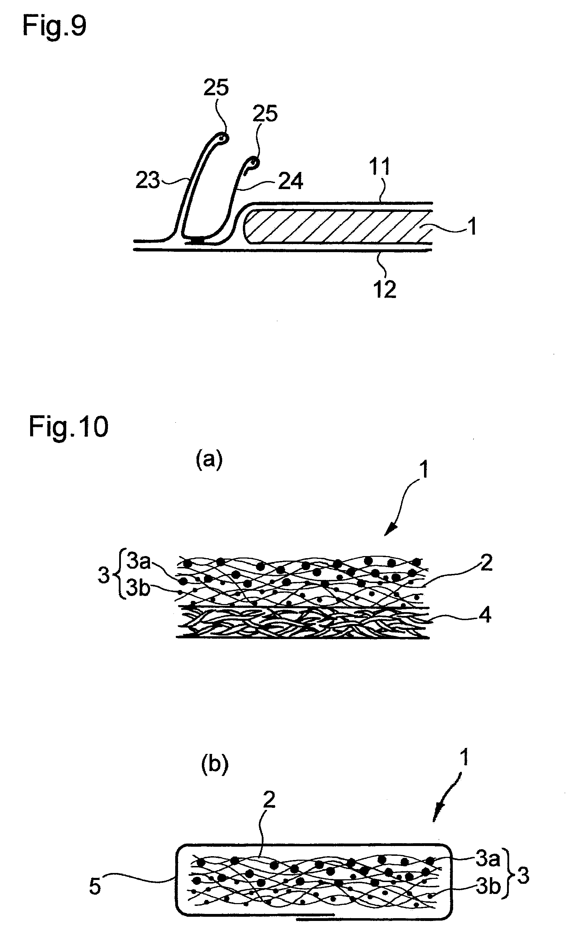

In the embodiment shown in Figs. 7 and 8, the barrier cuffs 23 and 24 are formed of respective single sheets. Otherwise, the two barrier cuffs 23 and 24 may be formed of a single sheet, such as a sheet of water repellent nonwoven fabric as shown in Fig. 9. In the embodiment of Fig. 9, the second barrier cuff 24 is formed of a side portion of a single sheet. The other portion of the sheet, adjacent to the second barrier cuff 24, is folded in two along a folding line extending in the longitudinal direction of the absorbent assembly 30 to form the first barrier cuff 23 having a double-paneled structure. An elastic thread 25 is disposed inside the folding line. Being double-paneled, the first barrier cuff 23 is less permeable to liquid. Therefore, if a liquid runs over the second barrier cuff 24 and is pooled between the first and second barrier cuffs 23 and 24, it hardly oozes out through the first barrier cuff 23.

Other embodiments of the present invention will be described with reference to Figs. 10(a) and 10(b). The description of the foregoing embodiments apply to these embodiments unless otherwise described. In Figs. 10(a) and 10(b), parts equivalent to those in Fig. 1 are indicated by the same numerical and alphabetical references.

In the embodiment shown in Fig. 10(a), an airlaid pulp layer 4 is disposed beneath the web 2. The airlaid pulp layer 4 may or may not contain a superabsorbent polymer. The airlaid fiber layer 4 is obtained by accumulating pulp fibers, or the airlaid fiber layer 4 is a nonwoven fabric made of pulp fibers. The airlaid fiber layer 4 obtained by accumulating pulp fibers may be any of airlaid fiber layers that have been commonly used as an absorbent member in conventional absorbent articles. The nonwoven fabric made of pulp fibers that can be used as the airlaid fiber layer 4 is exemplified by airlaid nonwoven fabric. Provided underneath the web 2, the airlaid fiber layer 4 serves as a temporary discharged-liquid reservoir. As a result, leakage is effectively prevented even when a body fluid is discharged at a high speed as in urination. To ensure the effect, it is preferred for the superabsorbent polymer embeddedly supported in the web 2 to be localized in the backsheet side of the web 2 as is shown in Fig. 10(a). When the superabsorbent polymer is incorporated into not only the web 2 but the airlaid pulp layer 4, the anti-leakage effect is further ensured. Moreover, the superabsorbent polymer in the web 2 acts as a moisture absorbent thereby to control a humidity rise inside the absorbent article while worn. As a result, the absorbent article hardly causes stuffiness.

Examples of the pulp as a fiber of the airlaid fiber layer 4 include natural pulp such as wood pulp and synthetic pulp made from synthetic resins such as polyethylene. Either one or both of the natural pulp and the synthetic pulp can be used. Use of natural pulp improves absorption capacity of the absorbent member 1 because natural pulp is, in general, a material having high liquid absorptivity. On the other hand, since synthetic pulp has properties of melting on being heated to a given temperature, the airlaid fiber layer 4 containing synthetic pulp, an absorbent core 5 containing synthetic pulp or the absorbent member 1 containing synthetic pulp may be heated to melt the synthetic fiber thereby to increase the strength of the absorbent member as a whole. When in using natural pulp, the airlaid fiber layer 4 is in most cases a layer obtained by accumulating pulp fibers. When in using synthetic pulp, the airlaid fiber layer 4 is in most cases a nonwoven fabric such as an airlaid nonwoven fabric.

The airlaid pulp layer 4 may contain, in addition to the above-described pulp fibers, natural or (semi)synthetic hydrophilic staple fibers of rayon, cotton, Lyocell, Tencel, cellulose acetate, polyvinyl alcohol fiber, acrylic fiber, and so forth.

In the embodiment shown in Fig. 10(b), the web 2 containing the superabsorbent polymer 3 is wrapped in a fiber sheet 5. In this embodiment, the fiber sheet 5 is a single sheet. The fiber sheet 5 covers the upper surface of the web 2, wraps around both the lateral sides of the web 2, and meets and overlaps with itself at the lateral middle of the lower surface of the web 2 by both the lateral side of the fiber sheet 5 to be rolled toward the lower surface of the web2. Thus, the fiber sheet 5 covers the lower surface of the laminate.

Wrapping the web 2 with the fiber sheet 5 effectively prevents the superabsorbent polymer 3 in the web 2 from extremely moving or falling off and improves the handling properties of the absorbent member 1 as an independent unitary member that can easily be transported. Being so wrapped in, the web can be trimmed or cut out with ease to provide an absorbent member of any desired shape according to the shape of an absorbent article.

The fiber sheet 5 is of an appropriate material having strength enough to prevent fall-off of the superabsorbent polymer and causing no hindrance to permeation of a discharged body fluid. The fiber sheet 5 is made up of substantially non-water absorbing fibers. The fiber sheet 5 is preferably formed of nonwoven fabric. The nonwoven fabric may be hydrophilized or perforated if needed. A slit may be cut in the nonwoven fabric. The nonwoven fabric can be made of single-component fibers of thermoplastic resins, such as polyethylene, polypropylene, and polyethylene terephthalate, or composite fibers containing two or more of these resins. Examples of the nonwoven fabrics are thermal-bonded nonwoven fabrics, spun-bonded nonwoven fabrics, spunbonded-meltblown-spunbonded nonwoven fabrics, spunbonded-meltblown-meltblown-spunbonded nonwoven fabrics, needle punched nonwoven fabrics, hydroentangled nonwoven fabrics, and airlaid nonwoven fabrics.

The present invention is not limited to the foregoing embodiments. For example, while in the embodiment shown in Fig. 1, the larger size polymer 3a is localized in the topsheet side, with the smaller size polymer 3b being localized in the backsheet side, the relation may be reversed. That is, the polymer in the backsheet side of the web 2 may be greater in size than that in the topsheet side. By distributing larger size polymer 3a in the backsheet side, liquid distribution occurs deeper in the absorbent member, which makes the absorbent member capable of receiving a large amount of urine. Since the smaller polymer particles in the topsheet side absorb fast, little liquid is allowed to remain in the topsheet to give a wearer a feeling of dryness. To develop these effects, it is preferred to prevent the smaller size polymer localized in the topsheet side from causing gel blocking thereby to secure rapid passage of liquid. From this viewpoint, it is preferred to use a superabsorbent polymer having high absorption capacity and high gel strength. For example, it is preferred to use the above-described superabsorbent polymer that has a liquid permeation rate of 150 to 1500 g/min under a load of 0.6 kPa.

It is more preferred to use a superabsorbent polymer having a high liquid permeation rate under a load of 2.0 kPa. The load of 2.0 kPa practically corresponds to the body pressure imposed to an absorbent member while worn. When a flow rate of urination is high as with the case of adult wearers or when the absorbent member is designed to have a reduced thickness, it is preferred to use a superabsorbent polymer having a liquid permeation rate of 30 to 300 ml/min, more preferably 32 to 200 ml/min, even more preferably 35 to 100 ml/min. If the liquid permeation rate is less than 30 ml/min, the superabsorbent polymer particles swollen with liquid to saturation are liable to stick to one another under load and obstruct passage of liquid (i.e., gel blocking). The higher the liquid permeation rate, the more preferred for preventing gel blocking from occurring. Where the liquid permeation rate exceeds 300 ml/min, however, the flow of the liquid in the absorbent member is too fast to catch up for immobilization, which can cause leakage, particularly when a large amount of excrement is discharged at a time or when the article is worn by older babies. In general, to increase the liquid permeation rate means to increase the degree of crosslinking of the superabsorbent polymer, which results in decreasing absorptive capacity of the superabsorbent polymer per unit weight. This leads to necessity to use an increased amount of the superabsorbent polymer. From all these considerations, the upper limit of the liquid permeation rate shall be decided.

A concrete method of measuring liquid permeation rate under a load of 2.0 kPa is described, e.g., in JP 2003-235889A, para. [0005]. In the present invention, the liquid permeation rate measurement is carried out by changing the sample weight from 0.200 g as specified in the above publication to 0.32 g. More specifically, the liquid permeation rate is measured according to the following procedures.

Method of measuring liquid permeation rate under 2.0 kPa load A filtration cylinder (inner diameter: 25.4 mm) equipped with a metal mesh (mesh size: 150 $\mu$m) and a narrow tube (inner diameter: 4 mm; length: 8 cm) with cock (inner diameter: 2 mm) is prepared. The cylinder with the tube closed with the cock is vertically held, and 0.32 g of a sample having a particle size adjusted to 850 to 150 $\mu$m is put therein. Then, 50 ml of 0.9 wt% physiological saline is poured in the cylinder. After allowing the cylinder to stand for 30 minutes from the start of pouring the physiological saline, a circular rod weighing 21.2 g and having attached to one end thereof a metal mesh having a mesh size of 150 $\mu$m and a diameter of 25 mm is inserted in the filtration cylinder until the metal mesh comes into contact with the sample. One minute later, a 77.0 g weight is attached to the circular rod to apply the appointed load to the sample. After the cylinder is left to stand for an additional 1 minute period, the cock is opened, and the time T1 (sec) required for the liquid level of the saline to drop from the 40 ml mark to the 20 ml mark is measured. The liquid transit time is calculated according to equation below using the thus measured time T1 (sec). In the equation, T0 is the time measured with no sample in the filtration cylinder.

$$\text{Liquid permeation rate (ml/min)} = 20\times60/(T1\text{-}T0)$$

For more detailed account of the method of liquid permeation rate measurement, refer to JP 2003-235889A, paras. [0008] and [0009]. The measuring equipment is shown in Figs. 1 and 2, *ibid.*

In order to decrease the packing density of the superabsorbent polymer as much as possible, it is preferred to reduce the amount of the superabsorbent polymer to be used. In this connection, the superabsorbent polymer preferably has a physiological saline absorption of 30 g/g or more, more preferably 30 to 50 g/g, measured by a centrifugal dewatering method. The physiological saline absorption measurement by a centrifugal dewatering method is carried out as follows. A superabsorbent polymer weighing 1 g is swollen with 150 ml of physiological saline over 30 minutes, put in a 250 mesh nylon bag, and dewatered using a centrifuge at 143 G (800 rpm) for 10 minutes. The gloss weight of the dewatered bag and the contents was measured, from which the absorption (g/g) by a centrifugal dewatering method is calculated according to equation:

Water absorption by centrifugal dewatering method = (gloss weight after dewatering –

weight of nylon mesh bag – dry weight of superabsorbent polymer – weight of liquid retained by nylon mesh bag)/dry weight of superabsorbent polymer

Any of the aforementioned superabsorbent polymers can be used with no particular restriction in each of the foregoing embodiments. In particular, a superabsorbent polymer having a desired liquid permeation rate can be obtained by adopting a reverse phase polymerization process using the anionic surface active agent proposed in commonly assigned Japanese patent 2721658 as a dispersing agent.

The absorbent article according to the present invention which includes an absorbent member having a continuos fiber web has a sparse structure containing large interfiber spaces compared with a conventional absorbent member made mainly of fluff pulp. Therefore, the absorbent member of the present invention has good liquid permeability. When the superabsorbent polymer is slow to absorb a liquid, it is likely to happen that a liquid fails to be sufficiently absorbed by the absorbent member because it passes through the absorbent member before being absorbed by the superabsorbent polymer. Taking this into consideration, it is desirable for the superabsorbent polymer in the web to have a sufficiently high absorption rate, whereby the liquid is retained in the absorbent member without fail. The absorption rate of the superabsorbent polymer is generally represented in the art by the value obtained by the demand wettability (DW) method. An absorption rate (ml/(0.3 g.30 sec)) by the DW method can be measured with a DW tester generally known for carrying out the DW method. In some detail, with the liquid levels of physiological saline being equal, 0.3 g of a superabsorbent polymer to be measured is scattered on a mount for scattering polymer particles (diameter: 70 mm; No. 1 glass filter having placed thereon No. 2 filter paper), and the water absorption after 30 seconds is gauged by reading the scale on the buret indicating a drop of the liquid level of physiological saline (the water absorption at the time of scattering the polymer is taken zero). The amount of absorption as measured is taken as an absorption rate. The absorption rate can be designed by selecting the shape, size, bulk density, degree of crosslinking and so on of the superabsorbent polymer particles.

In the embodiments of the present invention in which the absorbent member has a pulp content of 0 to 30% by weight, it is preferred to use a superabsorbent polymer having an absorption rate of 2 to 10 ml/(03 g.30 sec), more preferably 4 to 8 ml/(0.3 g.30 sec), measured by the DW method. In the production of conventional absorbent members made mainly of fluff pulp, the use of a superabsorbent polymer having such a high absorption rate has been avoided for fear of inducing gel blocking which can lead to leakage. In the present embodiment, since the web has a sparse structure, the absorbent member exhibits good properties of wicking liquid into the web and lets the wicked liquid pass in the web at a high speed. As a result, the superabsorbent polymer having such a high rate of absorption hardly causes gel blocking and, on the contrary, effectively prevents leakage. In the present invention, the term "weight of an absorbent member" is intended to include the weight of a sheet wrapping an absorbent member.

While the present invention has been described with reference to its preferred embodiments, the invention is not construed as being limited to the embodiments. For example, the absorbent member 1 shown in Fig. 10(a) may be entirely wrapped in a fiber sheet 5 as shown in Fig. 10(b).

Examples

The present invention will now be shown in greater detail by way of Examples, but it should be understood that the invention is not deemed to be limited thereto.

Example 1

A tow of crimped, continuous cellulose acetate fibers was prepared. The individual continuous fibers had a thickness of 2.1 dtex, and the total linear density of the tow was 25,000 dtex. The tow was fed under tension and opened in an air opening apparatus. The opened web was passed between a roller having a large number of discs arrayed around its periphery in an axial direction at a given interval and a smooth anvil roller, between which the web was combed. The width of the web was adjusted to 100 mm. The running speed of the web was slowed down, and the web was transferred onto a vacuum conveyor. The web on the vacuum conveyor was released from the tension to develop the crimp. The continuous fibers of the web had a crimp percentage of 30%, and the number of the crimps per centimeter was 15. The fiber interstices in the web were thus broadened thereby to help a superabsorbent polymer enter and to make the web bulkier thereby to improve polymer supporting capability. In this state, a superabsorbent polymer was spread on the web and embeddedly supported therein. The web was wrapped in tissue paper having a grammage of 16 g/m$^2$ to make an absorbent member. The adhesion between the upper and lower surface of the web and the tissue paper was with 5 g/m$^2$ of a hot melt adhesive applied thereto. The weights of the web and the superabsorbent polymer were 26 g/m$^2$ and 260 g/m$^2$, respectively.

An air-through nonwoven fabric weighing 25 g/m$^2$ was used as a topsheet. The air-through nonwoven fabric was made of linear low density polyethylene sheath/polypropylene core composite fiber (thickness: 2.1 dtex; having been treated with a surface active agent to have liquid permeability). A composite of a porous film weighing 20 g/m$^2$ and a spun-bonded polypropylene nonwoven fabric weighing 20 g/m$^2$, bonded with 1.5 g/m$^2$ of a hot melt adhesive, was used as a backsheet. The porous film was produced by blown-film extruding a uniform mixture of 100 parts by weight of linear low density polyethylene (density: 0.925 g/cm$^3$), 150 parts by weight of calcium carbonate, and 4 parts by weight of an ester compound as a third component and longitudinally stretching the blown film to double the length. A disposable diaper was assembled in otherwise the same manner as commonly used in the manufacture of disposable diapers. The absorbent member was disposed with the orientation direction of the web coinciding with the longitudinal direction of the diaper.

Example 2

A web having a superabsorbent polymer embeddedly supported therein was obtained in the same manner as in Example 1, except for changing the amount of the superabsorbent polymer to be spread to 110 g/m$^2$. Separately, 100 parts by weight of opened fluff pulp and 100 parts by weight of a superabsorbent polymer were uniformly mixed in an air stream and accumulated to obtain an airlaid fiber layer weighing 300 g/m$^2$. The resulting airlaid fiber layer and the above-prepared superabsorbent polymer-containing web were superposed on each other with 5 g/m$^2$ of a hot melt adhesive applied therebetween to obtain a laminate. The resulting laminate was wrapped in tissue paper having a grammage of 16 g/m$^2$ to obtain an absorbent member. The adhesion between the upper and lower surface of the laminate and the tissue paper was with 5 g/m$^2$ of a hot melt adhesive applied thereto. A disposable diaper was assembled in otherwise the same manner as in Example 1.

Example 3

A disposable diaper was obtained in the same manner as in Example 1, except that a sublayer sheet formed of air-through nonwoven fabric was disposed between the absorbent member and the topsheet. The air-through nonwoven fabric had a weight of 30 g/m$^2$ and was made of modified polyethylene terephthalate sheath/polyethylene terephthalate core composite fiber (thickness: 3.4 dtex; having been treated with a surface active agent to have liquid permeability).

Example 4

A disposable diaper was obtained in the same manner as in Example 2, except for preparing a laminate having an average weight of 30 g/m$^2$ from a tow having a total liner density of 17,000 dtex and composed of continuous fibers having a thickness of 6.7 dtex, a crimp percentage of 24%, and a number of crimps of 10/cm.

Comparative Example 1

A hundred parts by weight of opened fluff pulp and 100 parts by weight of a superabsorbent polymer were uniformly mixed in an air stream and accumulated to obtain a mixed aggregate weighing 520 g/m$^2$. The weights of the fluff pulp and the superabsorbent polymer were both 260 g/m$^2$. The resulting mixed aggregate was wrapped in tissue paper having a grammage of 16 g/m$^2$ to make an absorbent member. The adhesion between the mixed aggregate and the tissue paper was with 5 g/m$^2$ of a hot melt adhesive sprayed thereto. The absorbent member thus obtained had an overall weight of 562 g/m$^2$ and a thickness of 4.3 mm. A disposable diaper was assembled in otherwise the same manner as in Example 1.

Comparative Example 2

An absorbent member was prepared in the same manner as in Comparative Example 1, except for making a mixed aggregate weighing 300 g/m$^2$ (150 g/m$^2$ of fluff pulp, and 150 g/m$^2$ of the superabsorbent polymer). The absorbent member had an overall weight of 342 g/m$^2$ and a thickness of 2.7 mm. A disposable diaper was assembled in otherwise the same manner as in Example 1.

Comparative Example 3

In an attempt to make a mixed aggregate weighing 375 g/m$^2$, 100 pats by weight of opened fluff pulp and 200 pats by weight of a superabsorbent polymer were mixed in an air stream and accumulated. However, the resulting mixed aggregate failed to provide a laminate due to fall-off of the superabsorbent polymer.

Comparative Example 4

A superabsorbent polymer was spread on the same tow-opened web as in Example 1 to a weight of 260 g/m$^2$ to obtain a web, except that the web was not relaxed from tension and therefore with no crimp developed in this stage. The spread superabsorbent polymer was found remaining on the web without being embeddedly supported in the web. The web was wrapped in tissue paper having a grammage of 16 g/m$^2$ to obtain an absorbent member. The adhesion between the upper and lower surface of the laminate and the tissue paper was with 10 g/m$^2$ of a hot melt adhesive. A disposable diaper was made in otherwise the same manner as in Example 1.

Evaluation of performance

The absorbent member of each of the disposable diapers obtained in Examples and Comparative Examples was measured for absorption capacity and amount of rewet and evaluated for feeling of dryness, structural stability, and flexibility in accordance with the following methods. The results obtained are shown in Table 2 below.

(1) Absorption capacity

The absorbent member was fixed to an inclined plate set at 45°. A given amount of physiological saline was poured at a given time interval at a position 200 mm below the upper end of the absorbent member. The total amount of physiological saline that had been poured until it began to leak from the lower end of the absorbent member was compared. Taking the absorption capacity of Comparative Example 1 as 1.0, the results were expressed relatively by calculation using equation:

$$\text{Absorption capacity (relative)} = \text{absorption capacity of sample/absorption capacity of Comparative Example 1}$$

(2) Amount of rewet and feeling of dryness

DD & C Red No. 1 was added to physiological saline in a concentration of 50 ppm (0.5 g per 10 liters of physiological saline). The colored saline weighing 160 g was poured on the widthwise middle portion 150 mm below the frontal waist edge of the disposable diaper by use of a funnel. Ten minutes after completion of the pouring, a stack of ten sheets of filter paper No. 4A available from Advantech Tokyo Kaisha, Ltd. was placed on the wetted portion, and a load of 3.43 kPa was applied thereon for 2 minutes to have the filter paper absorb the saline. The filter paper was weighed, and the weight gain was taken as the amount of rewet. Measurement was made in triplicate.
Before the measurement of amount of rewet, 10 mothers were asked to observe the surface condition of the diaper after absorption and speak of their impression about dryness of the diapers. They were asked to judge the dryness using Comparative Example 1 as a benchmark. The dry feeling of the diaper was rated from the mothers' impression and the amount of rewet according to the following criteria.

A: 5 or more mothers answer that the diaper has a feeling of dryness equal to or more than that of Comparative Example 1, and the amount of rewet is 0.2 g or less.

B: 5 or more mothers answer that the diaper has a feeling of dryness equal to that of Comparative Example 1, and the amount of rewet is more than 0.2 g and not more than 0.3 g.

C: Less than 5 mothers answer that the diaper has a feeling of dryness, or the amount of rewet is more than 0.3 g.

D: 5 or more mothers answer that the diaper has no feeling of dryness, and the amount of rewet is more than 0.4 g.

(3) Structural stability

(3-1) While dry

The absorbent member measuring 100 mm by 200 mm was cut along its lateral centerline to prepare a 100 mm x 100 mm test piece. The piece was given 20 shakes with an amplitude of 5 cm at a rate of one shake per second with the cut area down. The polymer particles fallen from the cut area were weighed. The polymer supporting properties of the absorbent member was rated based on the ratio of the fallen polymer to the total polymer as follows.

A: The ratio of the fallen polymer is within 10%.

B: The ratio of the fallen polymer is higher than 10% and not higher than 25%.

C: The ratio of the fallen polymer is higher than 25%.

(3-2) While wet

A 100 mm by 200 mm cut piece of the absorbent member was almost uniformly impregnated with 200 g of physiological saline and then gently lifted to see whether the absorbent member broke or not with the naked eye. Apart from the above evaluation, the fallen superabsorbent polymer was weighed. The weight was divided by a water retention per unit weight of the superabsorbent polymer previously determined by a centrifugal dewatering method to obtain the dry weight of the fallen superabsorbent polymer. The ratio of the fallen superabsorbent polymer was calculated therefrom relative to the total amount of the polymer having been supported. The amount of the superabsorbent polymer that had been supported was determined as follows. An absorbent member to be analyzed, the weight of which had been measured, was immersed in an aqueous ascorbic acid solution and exposed to sunlight for a sufficient period of time to dissolve the superabsorbent polymer completely, followed by washing with water. The decomposition of the superabsorbent polymer and washing of the absorbent member with water were repeated until the superabsorbent polymer was completely dissolved. The absorbent member was then dried and weighed. The weight of the dried absorbent member was subtracted from the weight of the absorbent member before the superabsorbent polymer decomposition to give the amount of the superabsorbent polymer that had been present in the absorbent polymer.

A: The ratio of the fallen polymer is 10% or lower. No structural destruction of the absorbent member is observed.

B: The ratio of the fallen polymer is higher than 10% and not higher than 25%. No structural destruction of the absorbent member is observed.

C: The ratio of the fallen polymer is higher than 25%, or structural destruction of the absorbent member is observed.

(4) Flexibility

Flexibility of the absorbent member was evaluated by the use of a handle-o-meter. The smaller the "handle" value as measured with a handle-o-meter, the easier to put on and the snugger the fit. Measurement with a handle-o-meter is carried out as follows in accordance with JIS L1096 (stiffness and softness measuring method). A specimen measuring 50 mm in the CD and 150 mm in the MD is placed on the platform having a 60 mm wide slot with the length perpendicular to the slot. The force required for a 2 mm thick penetrator blade to force the center of the specimen into the slot is read. In the present invention, a handle-o-meter HOM-3 available from Daiei Kagaku Seiki Co., Ltd. was used. The measurement was made on three points to obtain an average. The flexibility was rated as follows.

A: The "handle" is 2N or less.

B: The "handle" is more than 2N and not more than 4N.

C: The "handle" is more than 4N.

Table 2

| | | Example | | | | Comparative Example | | | |
|---|---|---|---|---|---|---|---|---|---|
| | | 1 | 2 | 3 | 4 | 1 | 2 | 3 | 4 |
| Thickness (mm) | | 2.1 | 2.2 | 2.4 | 1.9 | 4.3 | 2.7 | - | - |
| Weight of continuous fibers (g/m$^2$) | | 26 | 26 | 26 | 30 | 0 | 0 | 0 | 20 |
| Thickness of continuous fibers | | 2.1 | 2.1 | 2.1 | 6.7 | - | - | - | - |
| Weight of superabsorbent polymer in continuous fiber web (g/m$^2$) | | 260 | 110 | | 110 | - | - | - | 260 |
| Weight of pulp (g/m$^2$) | | 0 | 150 | 0 | 0 | 260 | 150 | 125 | - |
| Weight of superabsorbent polymer in pulp layer (g/m$^2$) | | | 150 | 260 | 150 | 260 | 150 | 250 | - |
| Sublayer between topsheet and absorbent member | | - | - | air-through nonwoven | - | - | - | - | - |
| Absorption capacity | | 1.3 | 1.3 | 1.0 | 1.0 | 1.0 | 0.6 | - | 0.4 |
| Amount of rewet (g) | | 0.28 | 0.20 | 0.12 | 0.15 | 0.21 | 0.40 | - | - |
| Structural stability | dry | A | A | A | A | A | A | C | C |
| | wet | A | A | A | A | A | C | - | C |
| Flexibility | | A | A | A | A | C | A | - | - |
| Note: In Comparative Example 3, only a part of evaluation was conducted because of a failure to obtain a satisfactory absorbent member.<br>In Comparative Example 4, the superabsorbent polymer moved during the absorption capacity measurement, resulting in a leakage. | | | | | | | | | |

As is apparent from the results in Table 2, the absorbent members of Examples have high absorption capacity and reduced rewet. They also prove to exhibit high structural stability and good flexibility.

Industrial Applicability

As described in detail, since the absorbent member of the invention contains a superabsorbent polymer whose particle size varies between the topsheet side and the backsheet side thereof, it has an increased absorption rate. The absorbent member can be designed to have a reduced thickness and a reduced weight per unit area while maintaining an absorption capacity equal to that of a conventional one. Furthermore, the structure of the absorbent member is hardly destroyed by active movement of a wearer.

**Claims**

1. An absorbent article comprising an absorbent member having a topsheet side and a backsheet side,
the absorbent member comprising a web of hydrophilic continuous fibers and a superabsorbent polymer embeddedly supported in the web, and
the superabsorbent polymer having its particle size varied between the topsheet side and the backsheet side of the web.

2. The absorbent article according to claim 1, wherein the superabsorbent polymer has a larger particle size in the topsheet side than in the backsheet side of the web.

3. The absorbent article according to claim 1, wherein the superabsorbent polymer has a larger particle size in the backsheet side than in the topsheet side of the web.

4. The absorbent article according to any one of claims 1 to 3, further comprising a sublayer sheet between the absorbent member and a topsheet.

5. The absorbent article according to any one of claims 1 to 4, wherein the superabsorbent polymer has a rate of liquid permeation of 150 to 1500 g/min under a load of 0.6 kPa.

6. The absorbent article according to any one of claims 1 to 5, further comprising a barrier cuff extending in the longitudinal direction of the absorbent article doubly provided on each lateral side portion thereof.

7. The absorbent article according to any one of claims 1 to 6, wherein the continuous fibers are crimped fibers having a crimp percentage of 10% to 90%.

8. The absorbent article according to any one of claims 1 to 7, wherein the continuous fibers are oriented in a planar direction of the absorbent member.

Fig.1

Fig.2

Fig.3

Fig.4

Fig.5

Fig.6

Fig.7

Fig.8

# Fig.9

# Fig.10

(a)

(b)

## INTERNATIONAL SEARCH REPORT

| | International application No. |
|---|---|
| | PCT/JP2006/305963 |

A. CLASSIFICATION OF SUBJECT MATTER
*A61F13/49*(2006.01), *A61F13/53*(2006.01), *A61F13/15*(2006.01), *A61F13/539* (2006.01), *A61F13/494*(2006.01), *A61F13/472*(2006.01)

According to International Patent Classification (IPC) or to both national classification and IPC

B. FIELDS SEARCHED

Minimum documentation searched (classification system followed by classification symbols)
A61F13/15-13/84

Documentation searched other than minimum documentation to the extent that such documents are included in the fields searched
Jitsuyo Shinan Koho          1922-1996   Jitsuyo Shinan Toroku Koho    1996-2006
Kokai Jitsuyo Shinan Koho    1971-2006   Toroku Jitsuyo Shinan Koho    1994-2006

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y<br>A | JP 2000-516864 A   (Kimberly-Clark Worldwide, Inc.),<br>19 December, 2000 (19.12.00),<br>Page 19, lines 1 to 11; page 20, lines 2 to 6<br>& US 5294478 A           & EP 636017 A | 1,2,4-8<br>3 |
| Y | JP 9-273037 A   (Chisso Corp.),<br>21 October, 1997 (21.10.97),<br>Par. Nos. [0008], [0026]<br>(Family: none) | 1,2,4-8 |
| Y | JP 2001-258933 A   (Kao Corp.),<br>25 September, 2001 (25.09.01),<br>Par. No. [0005]<br>(Family: none) | 5 |

| ☒ Further documents are listed in the continuation of Box C. | ☐ See patent family annex. |
|---|---|

\*    Special categories of cited documents:
"A"  document defining the general state of the art which is not considered   to be of particular relevance
"E"  earlier application or patent but published on or after the international filing date
"L"  document which may throw doubts on priority claim(s) or which is cited to establish the publication date of another citation or other special reason (as specified)
"O"  document referring to an oral disclosure, use, exhibition or other means
"P"  document published prior to the international filing date but later than the priority date claimed

"T"  later document published after the international filing date or priority date and not in conflict with the application but cited to understand the principle or theory underlying the invention
"X"  document of particular relevance; the claimed invention cannot be considered novel or cannot be considered to involve an inventive step when the document is taken alone
"Y"  document of particular relevance; the claimed invention cannot be considered to involve an inventive step when the document is combined with one or more other such documents, such combination being obvious to a person skilled in the art
"&"  document member of the same patent family

| Date of the actual completion of the international search<br>    11 April, 2006 (11.04.06) | Date of mailing of the international search report<br>    18 April, 2006 (18.04.06) |
|---|---|
| Name and mailing address of the ISA/<br>    Japanese Patent Office | Authorized officer |
| Facsimile No. | Telephone No. |

Form PCT/ISA/210 (second sheet) (April 2005)

**INTERNATIONAL SEARCH REPORT**

| International application No. |
|---|
| PCT/JP2006/305963 |

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 10-277092 A   (Daio Paper Corp.),<br>20 October, 1998 (20.10.98),<br>Par. No. [0008]<br>(Family: none) | 6 |
| Y | JP 2001-258930 A   (Oji Paper Co., Ltd.),<br>25 September, 2001 (25.09.01),<br>Par. No. [0007]<br>(Family: none) | 6 |
| Y | JP 2003-33398 A   (Clemson University Research Foundation),<br>04 February, 2003 (04.02.03),<br>Par. No. [0126]; Fig. 23<br>& US 005972505 A1       & EP 000596038 A | 8 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 57160457 A
- WO 2001034082 A
- JP 2001276125 A
- US 5865822 A
- JP 2003235889 A
- JP 2721658 B